# EUROPÄISCHE PATENTSCHRIFT

(11) **EP 0 759 926 B1**
(45) Veröffentlichungstag und Bekanntmachung des Hinweises auf die Patenterteilung: **19.08.1998**
(21) Anmeldenummer: 95916634.9
(22) Anmeldetag: 13.04.1995
(51) Int. Cl.: C07H 19/19, C12P 19/40

(54) **VERFAHREN ZUR HERSTELLUNG VON ARABINONUKLEOSIDEN**
PROCESS FOR PRODUCING ARABINO-NUCLEOSIDES
PROCEDE DE FABRICATION D'ARABINONUCLEOSIDES

(30) Priorität: 20.05.1994 DE 4418474
(43) Veröffentlichungstag der Anmeldung: 05.03.1997
(73) Patentinhaber: SCHERING AKTIENGESELLSCHAFT, 13342 Berlin (DE)
(72) Erfinder: HUMMEL-MARQUARDT, Heidi, D-59368 Werne (DE); KENNECKE, Mario, D-14193 Berlin (DE); WEBER, Alfred, D-14169 Berlin (DE); SCHMITZ, Thomas, D-10997 Berlin (DE); TILSTAM, Ulf, D-13359 Berlin (DE); NICKISCH, Klaus, D-12307 Berlin (DE)
(86) Internationale Anmeldenummer: EP9501343
(87) Internationale Veröffentlichungsnummer: WO9532212

(56) Entgegenhaltungen:
- GB-A- 1 159 290
- US-A- 4 055 718
- US-A- 4 212 941
- JOURNAL OF ORGANIC CHEMISTRY, Bd. 33, Nr. 1, Januar 1968 EASTON US, Seiten 432-434, MONTGOMERY J.A. AND HEWSON K. 'A Convenient Method for the Synthesis of 2-Fluoroadenosine' in der Anmeldung erwähnt

## Beschreibung

Die Erfindung betrifft ein Verfahren zur Herstellung von Arabinonukleosiden der allgemeinen Formel I 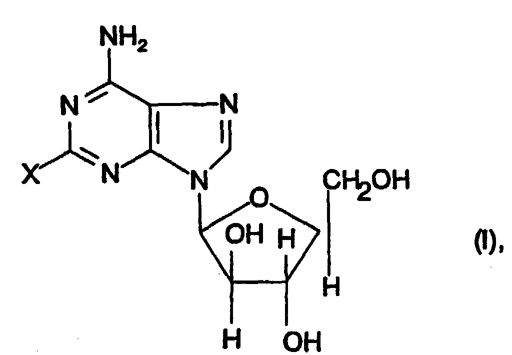 worin
X ein Wasserstoffatom oder ein Fluoratom darstellt, aus Triacetaten der allgemeinen Formel II 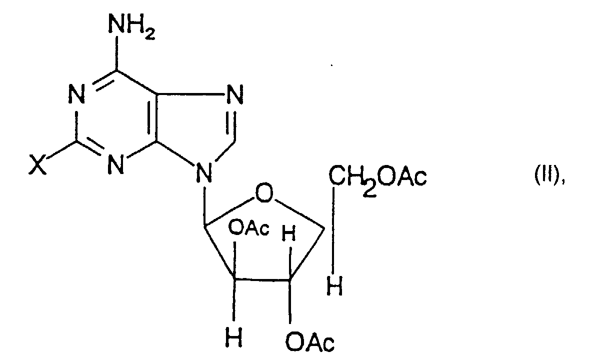 worin
X die oben genannte Bedeutung besitzt und die Gruppen
Ac jeweils Acetylgruppen bedeuten,
welches dadurch gekennzeichnet ist, daß man auf diese Verbindungen eine Esterase oder Lipase einwirken läßt.

Bekanntlich sind die Arabononukleoside der allgemeinen Formel I, das 9β-D-Arabinofuranosyl-9H-purin-6-amin (=Vidarabin) und das 9β-D-Arabinofuranosyl-2-fluor-9H-purin-6-amin (=Fludarabin) pharmakologisch wirksame Substanzen, die sich durch eine Antivirale und cytostatische Wirksamkeit auszeichnen (EP-A-317.728 und WO 9209604).

Nach dem bekannten Stand der Technik können diese Verbindungen aus den Triacetaten der allgemeinen Formel II hergestellt werden, indem man auf diese ethanolische Ammoniaklösung einwirken läßt (J.Org.Chem. 33. 1968.432ff; Can.J..Cem. 59, 1981, 2608ff und Nucleic Acids Symp. Ser. 1981, 9, 61ff). Dieses Verfahren ist aber nicht nur recht aufwendig, sondern hat darüber hinaus auch den Nachteil, daß insbesondere bei der Synthese von Fludarabin stark verunreinigte Verfahrensprodukte erhalten werden.

Demgegenüber ist das erfindungsgemäße Verfahren mit relativ geringem Aufwand durchführbar und liefert die gewünschten Verfahrensprodukte in hoher Reinheit.

Die enzymatische Hydrolyse der Triacetate der allgemeinen Formel II verläuft selbst bei hohen Substratkonzentrationen quantitativ. Dies ist für den Fachmann überraschend, da hinlänglich bekannt ist, daß Acetate mehrwertiger Alkohole enzymatisch oft nur partiell verseift werden (Tetrahedron 46, 1990, 6587-6611)

Das erfindungsgemäße Verfahren kann mit handelsüblichen Carbonsäure-Esterasen bzw. Lipasen durchgeführt werden. Solche sind beispielsweise Esterase aus Schweineleber der Firma Fluka AG; Ch-9470 Buche) mit ca. 130 U/mg Protein (1U entspricht der Enzymmenge, die bei 25°C und pH 8.0 1µmol Buttersäureethylester umsetzt),
Esterase aus Schweineleber der Firma Boehringer-Mannheim mit ca. 130 U/mg Protein, Esterase aus Schweineleber der Firma Sigma Corp. St. Luis USA mit ca. 230 U/mg Protein,
Acylase I aus Aspergillus melleus der Fa. Sigma Corp. mit ca. 0,5 U/mg Feststoff Lipase Typ II aus Schweinepankreas der Fa. Sigma Corp. mit ca. 110-220 U/mg Prot. (Substrat Olivenöl)
Lipase F7 aus Mucor sp. der Fa. Eurymatic Ltd., Cambridge
Zur Durchführung des erfindungsgemäßen Verfahrens wird das Substrat und Enzym in einer geeigneten Pufferlösung (Citratpuffer, Phosphatpuffer, Trispuffer etc.) gelöst und bei 30 bis 42°C geschüttelt oder gerührt, bis eine vollständige Umsetzung erreicht ist. Bei dieser Umsetzung werden normalerweise 1 bis 10 g Substrat und 1 bis 100 mg Enzym pro 1 eingesetzt. Nach erfolgter Umsetzung (die in einfacher Weise durch Chrommatographie wie HPLC- oder DC-Chromatographie ermittelt werden kann) kann die Isolierung des Verfahrensproduktes in einfacher Weise durch Einengen der Reaktionsmischung isoliert werden.

Um die Esterase mehrfach verwenden zu können, ist es zweckmäßig, diese in bereits bekannter Weise zu immolisieren (Tetrahedron 26 (no4) pp 407-410 (1985)). oder zur Durchführung der Reaktion eine handelsübliche immobilisierte Esterase (wie zum Beispiel die auf Eupergit® (immobilisierte Esterase aus Schweineleber der Firma Fluka AG) zu verwenden.

Die nachfolgenden Ausführungsbeispiele dienen zur näheren Erläuterung des erfindungsgemäßen Verfahrens.

### Beispiel 1

100 ml 0,25 molarer wässriger Tris-(hydroxymethyl)-aminomethan/HCl Puffer vom pH 8.7 wird mit 0,500 g (2',3',5'-Tri-O-acetyl-9β-D-arabinofuranosyl)-2-fluor-9H-purin-6-amin und 0,1 mg Schweineleber-Esterase (Boehringer Mannheim; 130 U/mg Protein) versetzt und 70 Stunden lang bei 37°C gerührt.

Nach HPLC ist die erhaltene Reaktionslösung praktisch frei von 9β-D-Arabinofuranosyl-2-fluor-9H-purin-6-aminmonoacetat oder -diacetat. Sie wird bei maximal 50°C Badtemperatur auf ca. 5 ml eingeengt, das ausgefallene Produkt abgesaugt, mit Wasser gewaschen und im Vakuum bei 100°C getrocknet. Man erhält so das 9β-D-Arabinofuranosyl-2-fluor-9H-purin-6-aminin quantitativerAubeute.

### Beispiel 2

Unter den Bedingungen des Beispiels 1 werden 0,500 g (2',3',5'-Tris-O-acetyl-9β-D-arabinofuranosyl)-2-fluor-9H-purin-6-amin und 0,1 mg Schweineleber-Esterase (Sigma, Chem. St. Luis, USA 230 U/mg Protein) umgesetzt, aufbereitet und man erhält ebenfalls in quantitativer Ausbeute das 9β-D-Arabinofuranosyl-2-fluor-9H-purin-6-amin.

### Beispiel 3

Unter den Bedingungen des Beispiels 1, jeodch unter Einsatz von 1,00 g (2',3',5'-Tris-O-acetyl-9β-D-arabinofuranosyl)-2-fluor-9H-purin-6-amin 200 Stunden lang inkubiert. Die Reaktionslösung wird aufbereitet wie im Beispiel 1 beschrieben und man erhält ebenfalls in quantitativer Ausbeute das 9β-D-Arabinofuranosyl-2-fluor-9H-purin-6-amin.

### Beispiel 4

a) Eupergit® (Fluka AG) wird im Verhältnis 10:1 mit Schweineleber-Esterase (Boehringer Mannheim; 130 U/mg Protein) und 1 molarem wässrigen Phosphatpuffer vom pH 7.5 gemischt und 3 Tage lang bei Raumtemperatur inkubiert. Dann wird das Produkt filtriert, mit Phosphatpuffer und destilliertem Wasser gewaschen und in 1 molarem Phosphatpuffer von pH 7.5, der 0,05% Benzoesäureethylester enthält, aufbewahrt.
b) 100 ml 0,5 molarer wässriger Tris-(hydroxyethyl)-aminomethan/HCl Puffer vom pH 7.5 werden mit 500 mg (2',3',5'-Tri-O-acetyl-9β-D-arabinofuranosyl)-2-fluor-9H-purin-6-amin und 0,5 mg gemäß Beispiel a hergestellter immobilisierter Schweineleber-Esterase versetzt und 100 Stunden lang bei 37°C inkubiert Nach dieser Zeit haben sich ca. 85% des Ausgangsmaterials umgesetzt.
   Das Immobilisat wird abgetrennt und kann dann bis zu 30mal wiederverwendet werden.

## Patentansprüche

1. Verfahren zur Herstellung von Arabinonukleosiden der allgemeinen Formel I 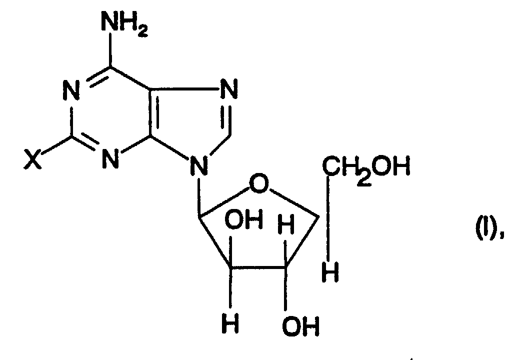 worin
X ein Wasserstoffatom oder ein Fluoratom darstellt, aus Triacetaten der allgemeinen Formel II 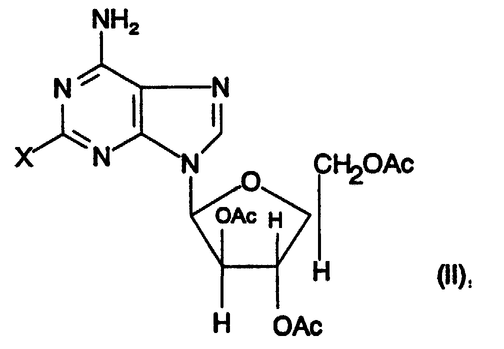 worin
X die oben genannte Bedeutung besitzt und die Gruppen
Ac jeweils Acetylgruppen bedeuten,
welches dadurch gekennzeichnet ist, daß man die diese Verbindungen eine Esterase oder Lipase einwirken läßt.

## Claims

1. Process for the preparation of arabino-nucleosides of the general formula I 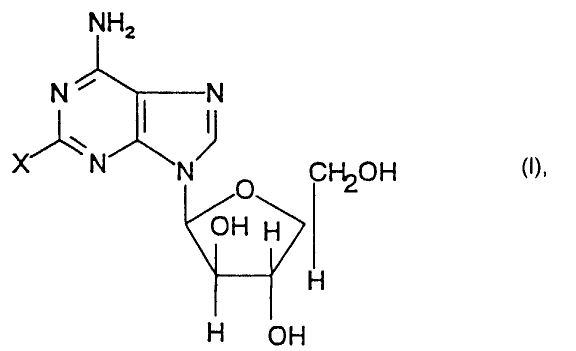 in which
X represents a hydrogen atom or a fluorine atom,
from triacetates of the general formula II 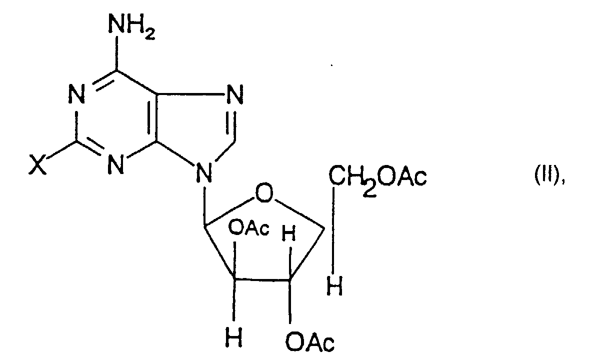 in which
X is as defined above, and
each of the groups Ac represents an acetyl group,
characterised in that an esterase or lipase is allowed to act on those compounds.

## Revendications

1. Procédé de préparation d'arabinonucléosides répondant à la formule générale I 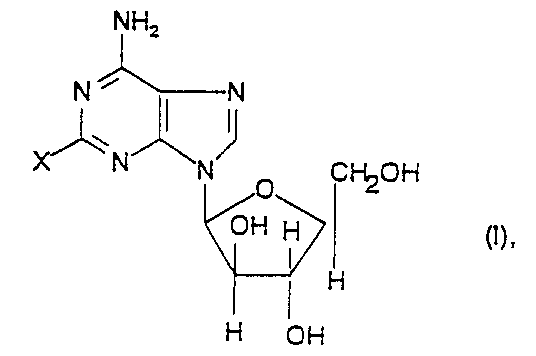 dans laquelle
X représente un atome d'hydrogène ou un atome de fluor, à partir de triacétates de la formule générale II 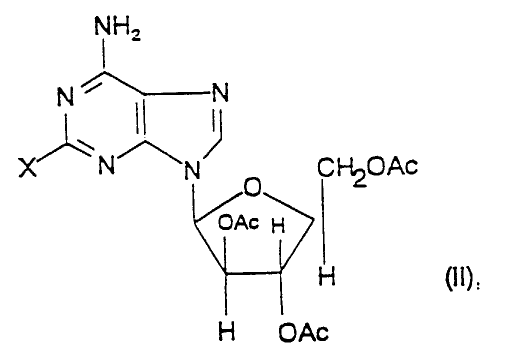 dans laquelle
X a la même signification que celle donnée ci-dessus et les groupes
Ac représentent chacun un groupe acétyle,
ce procédé étant caractérisé en ce qu'on fait agir sur ces composés une estérase ou lipase.
